(19) Europäisches Patentamt
European Patent Office
Office européen des brevets

(11) Veröffentlichungsnummer: **0 037 049**
**A1**

# (12) EUROPÄISCHE PATENTANMELDUNG

(21) Anmeldenummer: **81102165.8**

(22) Anmeldetag: **23.03.81**

(51) Int. Cl.³: **C 07 D 233/56**
C 07 D 249/08, A 61 K 31/41
A 61 K 31/415

(30) Priorität: **02.04.80 DE 3012770**

(43) Veröffentlichungstag der Anmeldung:
**07.10.81 Patentblatt 81/40**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Böshagen, Horst, Dr.**
**Wiesenstrasse 4**
**D-5657 Haan(DE)**

(72) Erfinder: **Regel, Erik, Ing.-grad.**
**Bergerheide 72a**
**D-5600 Wuppertal 1(DE)**

(72) Erfinder: **Büchel, Karl Heinz, Prof. Dr.**
**Dabringhausener Strasse 42**
**D-5093 Burscheid(DE)**

(72) Erfinder: **Plempel, Manfred, Dr.**
**Pahlkestrasse 5**
**D-5600 Wuppertal 1(DE)**

(54) **Biphenylyl-azolylethan-Derivate, Verfahren zu ihrer Herstellung und ihre Verwendung als Arzneimittel.**

(57) Biphenylyl-azolylethan-derivate der allgemeinen Formel

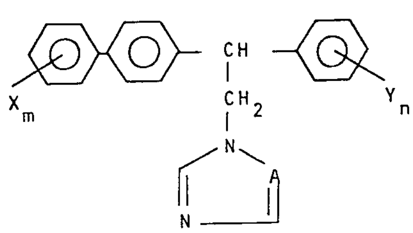

(1)

in welcher
A für die CH-Gruppe oder ein Stickstoffatom steht,
X für Halogen, Alkyl, Halogenalkyl, Alkoxy, Alkylthio, Nitro oder Cyano steht,
Y für Halogen, Alkyl, Halogenalkyl, Alkoxy, Alkylthio, Nitro, Cyano oder gegebenenfalls substituiertes Phenyl steht und

m und n für 0, 1, 2 oder 3 stehen,
deren physiologisch verträglichen Säureadditions Salze, ein Verfahren zu ihrer Herstellung und ihre Verwendung als Arzneimittel, insbesondere als Antimykotika.

EP 0 037 049 A1

BAYER AKTIENGESELLSCHAFT 5090 Leverkusen, Bayerwerk
Zentralbereich
Patente, Marken und Lizenzen Si/Th
Ia

Biphenylyl-azolylethan-Derivate, Verfahren zu ihrer Herstellung und ihre Verwendung als Arzneimittel

Die vorliegende Erfindung betrifft neue Biphenylylazolyl-ethan-Derivate, ein Verfahren zu ihrer Herstellung und ihre Verwendung als Arzneimittel, insbesondere als Antimykotika.

Es ist bereits bekannt geworden, daß Biphenylyl-azolylmethan-Derivate gute antimykotische Eigenschaften aufweisen (vergleiche DE-OS 24 61 406 bzw. die US-Patentschrift 4 118 487). Deren Wirkung ist jedoch nicht immer bei allen Pilzspezies voll befriedigend.

Es wurden neue Biphenylyl-azolylethan-Derivate der allgemeinen Formel

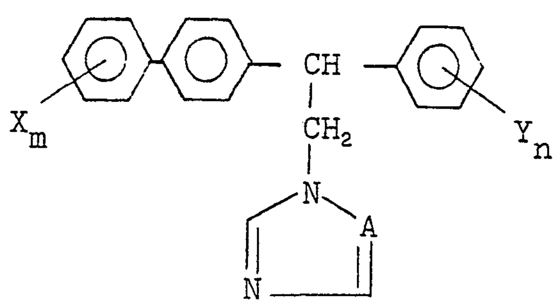

(I)

in welcher

A für die CH-Gruppe oder ein Stickstoffatom steht,

X für Halogen, Alkyl, Halogenalkyl, Alkoxy, Alkylthio, Nitro oder Cyano steht,

Y für Halogen, Alkyl, Halogenalkyl, Alkoxy, Alkylthio, Nitro, Cyano oder gegebenenfalls substituiertes Phenyl steht und

m und n für 0, 1, 2 oder 3 stehen,

und deren physiologisch verträglichen Säureadditions-Salze gefunden.

Weiterhin wurde gefunden, daß man die Biphenylyl-azolylethan-Derivate der Formel (I) erhält, wenn man Biphenylyl-azolylethylen-Derivate der Formel

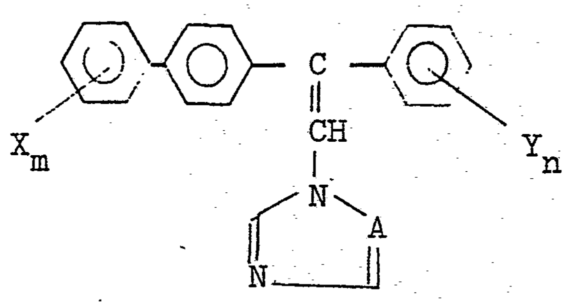

(II)

in welcher

A, X, Y, m und n die oben angegebene Bedeutung haben,

in üblicher Weise mit Wasserstoff in Gegenwart eines Katalysators und gegebenenfalls in Gegenwart eines Verdünnungsmittels hydriert.

Die neuen Biphenylyl-azolylethan-Derivate weisen starke antimykotische Eigenschaften auf. Dabei zeigen überraschenderweise die erfindungsgemäßen Verbindungen eine höhere Wirkung als die aus dem Stand der Technik bekannten Biphenylyl-azolylmethan-Derivate, welche chemisch und wirkungsmäßig naheliegende Verbindungen sind. Die erfindungsgemäßen Stoffe stellen somit eine Bereicherung der Pharmazie dar.

Die erfindungsgemäßen Stoffe sind durch die Formel (I) allgemein definiert. In dieser Formel steht X vorzugsweise für Halogen, geradkettiges oder verzweigtes Alkyl mit 1 bis 4 Kohlenstoffatomen, Halogenalkyl mit 1 bis 4 Kohlenstoff- und 1 bis 5 gleichen oder verschiedenen Halogenatomen, wobei als Halogene vorzugsweise Fluor und Chlor stehen; ferner vorzugsweise für Alkoxy und Alkylthio mit jeweils 1 bis 4 Kohlenstoffatomen, sowie Nitro und Cyano. Y steht vorzugsweise für die Reste, die bereits für X vorzugsweise genannt wurden, sowie für gegebenenfalls durch X substituiertes Phenyl. A und die Indizes m und n haben vorzugsweise die in der Erfindungsdefinition angegebene Bedeutung.

Ganz besonders bevorzugt sind diejenigen Verbindungen der Formel (I), in denen X für Fluor, Chlor oder Brom; für Methyl, Ethyl, Isopropyl oder tert.-Butyl; für Trifluormethyl; für Methoxy, Ethoxy, Methylthio oder Ethylthio; sowie für Nitro oder Cyano steht; Y für die für X genannten Reste steht sowie für gegebenenfalls durch Fluor, Chlor, Methyl, tert.-Butyl, Trifluormethyl, Nitro oder Cyano substituiertes Phenyl steht; A sowie die Indizies m und n die in der Erfindungsdefinition angegebene Bedeutung haben.

Im einzelnen seien außer den bei den Herstellungsbeispielen angegebenen Verbindungen die folgenden Verbindungen der allgemeinen Formel (I) genannt:

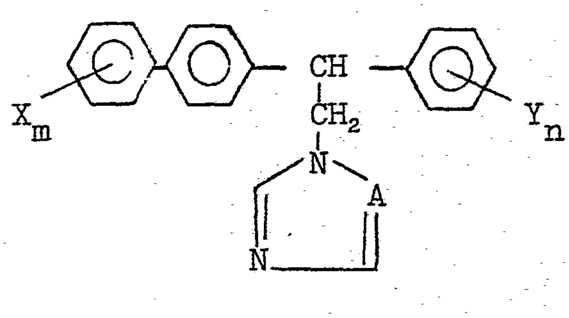

| $X_m$ | $Y_n$ | A |
|---|---|---|
| - | 2-Cl | N |
| - | 3-F | N |
| - | 3-Cl | N |
| - | - | CH |
| - | 4-Cl | CH |
| - | $3-CH_3$ | CH |
| - | $4-C(CH_3)_3$ | CH |
| 4-Cl | 3-Cl | CH |
| 4-Cl | 2-Cl | CH |
| - | $3-CF_3$ | CH |
| - | $2,5-Cl_2$ | CH |
| - | 4-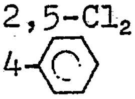 | CH |
| - | 4-Br | CH |
| - | $4-Cl, 3-NO_2$ | CH |
| - | $3-NO_2$ | CH |

| $X_m$ | $Y_n$ | A |
|---|---|---|
| - | - | N |
| - | 4-Cl | N |
| - | $3-CH_3$ | N |
| - | $4-C(CH_3)_3$ | N |
| 4-Cl | 3-Cl | N |
| 4-Cl | 2-Cl | N |
| - | $3-CF_3$ | N |
| - | $2,5-Cl_2$ | N |
| - | 4-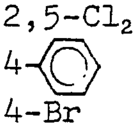 | N |
| - | 4-Br | N |
| - | $4-Cl,3-NO_2$ | N |
| - | $3-NO_2$ | N |

Verwendet man beispielsweise 1-(4-Biphenylyl)-1-(2-chlorphenyl)-2-(imidazol-1-yl)-ethan und Wasserstoff als Ausgangsstoffe, so kann der Reaktionsablauf durch das folgende Formelschema wiedergegeben werden:

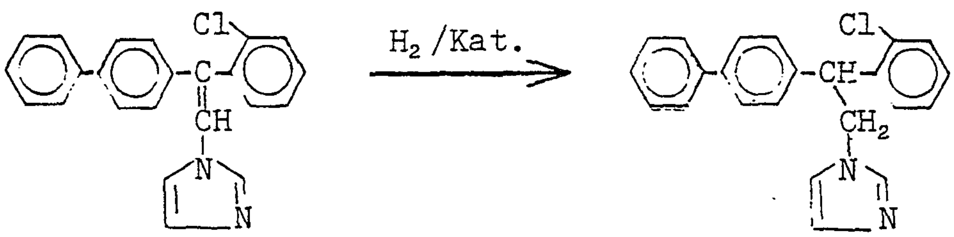


Die für das erfindungsgemäße Verfahren als Ausgangsstoffe zu verwendenden Biphenylyl-azolylethylen-Derivate sind durch die Formel (II) allgemein definiert. In dieser Formel stehen A, X, Y und die Indizes m und n vorzugsweise für diejenigen Bedeutungen, die bereits im Zusammenhang mit der Beschreibung der erfindungsgemäßen Stoffe der Formel (I) vorzugsweise für diese Substituenten genannt wurden.

Die Biphenylyl-azolylethylen-Derivate der Formel (II) sind noch nicht bekannt. Sie werden erhalten, indem man Hydroxyethyl-azole der Formel

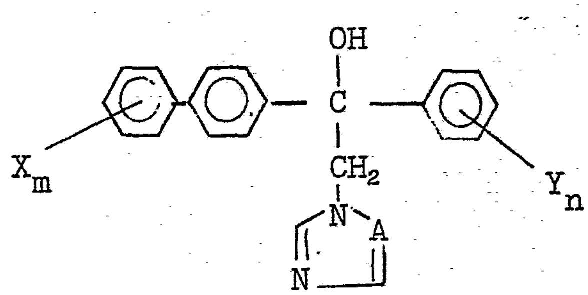

(III)

in welcher

A,X,Y,m und n die oben angegebene Bedeutung haben,

mit einem wasserbindenden Mittel gegebenenfalls in Gegenwart eines Verdünnungsmittels umsetzt.

Als wasserbindende Mittel seien vorzugsweise genannt: Säureanhydride, wie Essigsäureanhydrid; Halogenide von Sauerstoffsäuren,wie Thionylchlorid; sowie Isocyanate, wie Methylisocyanat.

Als Verdünnungsmittel kommen für diese Umsetzung vorzugsweise inerte organische Lösungsmittel infrage. Hierzu gehören vorzugsweise chlorierte Kohlenwasserstoffe, wie Chloroform oder Methylenchlorid.

Die Reaktionstemperaturen können bei der Durchführung dieser Umsetzung in einem größeren Bereich variiert werden. Im allgemeinen arbeitet man zwischen 0 und 120°C, vorzugsweise zwischen 20 und 100°C.

Bei der Durchführung dieser Umsetzung arbeitet man vorzugsweise in molaren Mengen bzw. mit einem Überschuß an wasserbindendem Mittel. Die Isolierung der Verbindungen der Formel (II) erfolgt nach üblichen Methoden (vergleiche auch die Herstellungsbeispiele).

Die Hydroxyethyl-azole der Formel (III) sind ebenfalls noch nicht bekannt; sie sind jedoch Gegenstand einer eigenen älteren Anmeldung, die noch nicht veröffentlicht ist (vergleiche die deutsche Patentanmeldung P 28 51 116 vom 25.11.1978 [LeA 19 273]). Sie können erhalten werden, indem man die entsprechenden Hydroxyethyl-halogenide mit Imidazol oder Triazol, gegebenenfalls in Gegenwart eines Säurebindemittels, wie vorzugsweise einem Ueberschuß an Azol, oder in Form eines ihrer Alkalimetallsalze, wie sie beispielsweise durch Behandlung mit Natriummethylat in einem geeigneten Lösungsmittel erhalten werden, gegebenenfalls in Gegenwart eines inerten organischen Lösungsmittels, wie z.B. Dimethylformaid, bei Temperaturen zwischen 30 und 120°C umsetzt.

Die Hydroxyethyl-azole der Formel (III) können auch nach einem weiteren Verfahren erhalten werden, welches ebenfalls Gegenstand einer eigenen älteren Anmeldung ist, die noch nicht veröffentlicht ist (vergleiche die deutsche Patentanmeldung P 29 12 288 vom 28.3.1979 [LeA 19 527]), indem man Oxirane der Formel

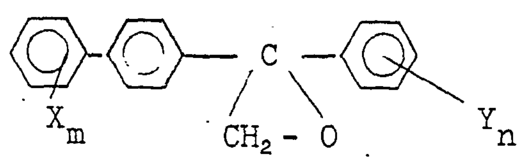 (IV)

in welcher

X,Y,m und n die oben angegebene Bedeutung haben,

mit Imidazol oder Triazol in Gegenwart eines Alkalialkoholats, wie z.B. Natriummethylat, und in Gegenwart eines inerten organischen Lösungsmittels, wie z.B. Dimethylformamid, bei Temperaturen zwischen 30 und 100°C umsetzt (vergleiche auch die Herstellungsbeispiele).

Die Oxirane der Formel (IV) sind noch nicht bekannt. Auch sie sind Gegenstand der erwähnten vorgängigen Anmeldung (vergleiche die deutsche Patentanmeldung P 29 12 288 vom 28.3.1979 [LeA 19 527], und können erhalten werden, indem man entsprechende Ketone entweder mit Dimethyloxosulfonium-methylid in an sich bekannter Weise in Gegenwart eines Verdünnungsmittels, wie beispielsweise Dimethylsulfoxid, bei Temperaturen zwischen 20 und 80°C umsetzt; oder mit Trimethylsulfonium-methylsulfat in an sich bekannter Weise in Gegenwart eines Zweiphasensystems und gegebenenfalls in Gegenwart eines Phasentransferkatalysators bei Temperaturen zwischen 0 und 100°C umsetzt (vergleiche auch die Herstellungsbeispiele).

Die erfindungsgemäße Hydrierung wird in Gegenwart eines Katalysators durchgeführt. Vorzugsweise wurden Edelmetall-, Edelmetalloxid- (bzw. Edelmetallhydroxid-) oder Raney-Katalysatoren verwendet, wie beispielsweise Platin, Platinoxid, Raney-Nickel und Palladiumoxid.

Als Verdünnungsmittel kommen für die erfindungsgemäße Hydrierung vorzugsweise inerte organische Lösungsmittel infrage. Hierzu gehören vorzugsweise Alkohole, wie Methanol und Ethanol; Nitrile, wie Acetonitril; Ester, wie Essigester; sowie Eisessig.

Die Reaktionstemperaturen können bei der erfindungsgemäßen Hydrierung in einem größeren Bereich variiert werden. Im allgemeinen arbeitet man zwischen 20 und 200°C, vorzugsweise bei 40 bis 150°C.

Die erfindungsgemäße Hydrierung wird vorzugsweise unter erhöhtem Druck durchgeführt. Im allgemeinen arbeitet man zwischen 1 und 200 atü, vorzugsweise bei 1 bis 150atü.

Bei der erfindungsgemäßen Hydrierung setzt man vorzugsweise auf 1 Mol der Verbindung der Formel (II) etwa 1 Mol Wasserstoff und etwa 0,1 Mol Katalysator ein. Die Isolierung der Endprodukte der Formel (I) erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maliensäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure. Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Verbindungen der Formel (I) und ihre Säureadditions-Salze weisen antimikrobielle, insbesondere starke antimykotische Wirkungen auf. Sie besitzen ein sehr breites Wirkungsspektrum in vitro, das Dermatophyten, Hefen, Pityrosporum ovale, Schimmelpilze und biphasische Pilze umfaßt. Sie können deshalb mit gutem Erfolg gegen Pilzinfektionen bei Mensch und Tier eingesetzt werden.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die oben genannten Erreger hervorgerufen werden.

Die erfindungsgemäßen Wirkstoffe sind gegen ein sehr breites Spektrum von Mikroorganismen wirksam. Mit ihrer Hilfe können z.B. Gram-negative und Gram-positive Bakterien und bakterienähnliche Mikroorganismen bekämpft sowie die durch diese Erreger hervorgerufenen Erkrankungen verhindert, gebessert und/oder geheilt werden.

Zur vorliegenden Erfindung gehären pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragges, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreit wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen,

Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylenzoat, Propylenglykol, 1,3-Butylenglykol, Dimethylformamid, Öle, insbesondere Baumwollsaatöle, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süßmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

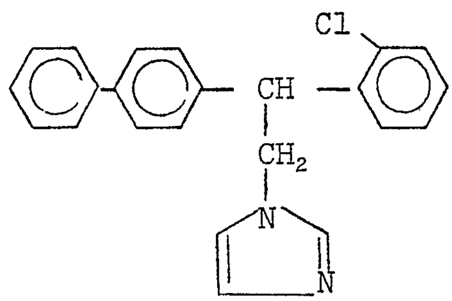

4,0g 1-(4-Biphenylyl)-1-(2-chlorphenyl)-2-(imidazol-1-yl)-ethylen werden in 100 ml Eisessig gelöst und mit Platinoxid als Katalysator 8 Stunden bei 60°C und 40 bar/$H_2$ hydriert. Anschließend wird das Lösungsmittel abdestilliert und der sirupöse Rückstand über Kieselgel 60-Merck chromatographiert (Laufmittel: Chloroform/Methanol = 10/0,5). Man erhält 3,4g (85 % der Theorie) 1-(4-Biphenylyl)-1-(2-chlorphenyl)-2-(imidazol-1-yl)-ethan vom Schmelzpunkt 86°C.

Herstellung der Vorstufen

(II-1)

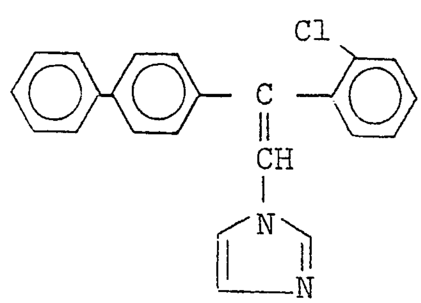

1,9g 1-(4-Biphenylyl)-1-(3-chlorphenyl)-2-(imidazol-1-yl)-ethan-1-ol werden in 30 ml Chloroform suspendiert und 1,4 ml Thionylchlorid zugesetzt. Die Mischung wird 18 Stunden bei 60°C gerührt. Dann wird die klare Lösung im Vakuum eingedampft, der Rückstand in 30 ml Chloroform aufgenommen und nach Zusatz von weiteren 1,4 ml Thionylchlorid erneut 18 Stunden bei 60°C gerührt. Das nach dem Abziehen des Lösungsmittels zurückbleibende kristalline

Rohprodukt wird aus Isopropanol umkristallisiert. Man erhält 16g (89 % der Theorie) 1-(4-Biphenylyl)-1-(2-chlorphenyl)-2-(imidazol-1-yl)-ethylen vom Schmelzpunkt 196°C.

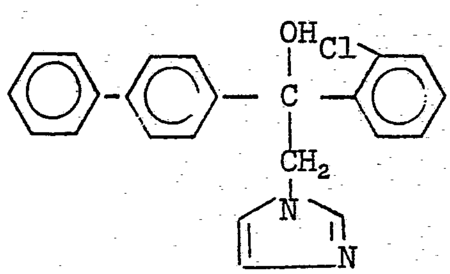

3,5g (0,065 Mol) Natriummethylat werden in 20 ml Methanol gelöst und mit 7,5g (0,11 Mol) Imidazol versetzt. Dazu wird eine Lösung von 15g (0,05 Mol) 2-(4-Biphenylyl)-2-(2-chlorphenyl)-oxiran in 75 ml Dimethylformamid getropft und das Reaktionsgemisch 1,5 Stunden auf 80°C erhitzt. Danach wird das Reaktionsgemisch auf Wasser gegossen, die entstehenden Kristalle werden abfiltriert und getrocknet. Man erhält 14,7 g (79 % der Theorie) 1-(4-Biphenylyl)-1-(2-chlorphenyl)-2-imidazol-1-yl-ethan-1-ol vom Schmelzpunkt 222°C.

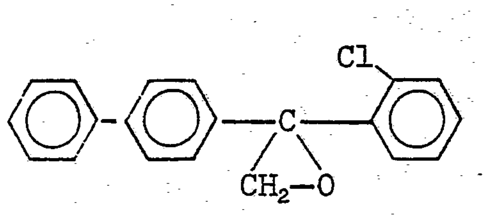

2,7g (0,09 Mol) 80%-iges Natriumhydrid und 19,8g (0,09 Mol) Trimethyloxosulfoniumjodid werden innerhalb 20 Minuten mit 90 ml Dimethylsulfoxid versetzt. Nach beendeter Wasserstoffentwicklung wird eine Lösung von 22g (0,075 Mol) 2-Chlor-4'-phenyl-benzophenon in 60 ml Dimethylsulfoxid zugetropft und 1 Stunde bei 50°C nachgerührt. Das erkaltete Reaktionsgemisch wird mit 200 ml Wasser versetzt und mit Ether ausgeschüttelt. Die Etherlösung wird mit Wasser gewaschen, über Natriumsulfat getrocknet und durch Abdestillieren des Lösungsmittels im Vakuum eingeengt. Der Rückstand wird aus Diisopropylether umkristallisiert. Man erhält 15g (65 % der Theorie) 2-(4-Biphenylyl)-2-(2-chlorphenyl)-oxiran vom Schmelzpunkt 70°C.

Entsprechend Beispiel 1 können die in der folgenden Tabelle 1 aufgeführten Verbindungen der Formel (I) erhalten werden:

Tabelle 1

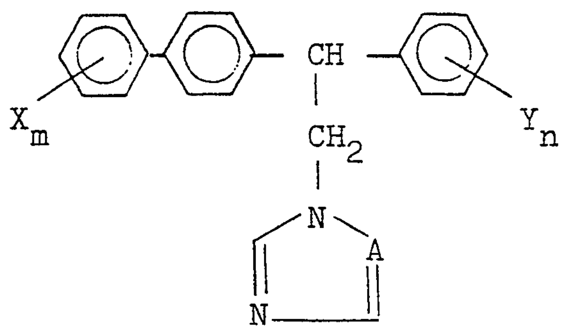

(I)

| Bsp. Nr. | $X_m$ | $Y_n$ | A | Schmelzpunkt(°C) |
|---|---|---|---|---|
| 2 | 4-Cl | 2-Cl | CH | 188 |
| 3 | - | 2-F | CH | Harz |
| 4 | - | 3-Cl | CH | Oel |
| 5 | - | 2-Cl | CH | 282(x1/2 NDS) |
| 6 | - | 2-F | CH | 297(x1/2 NDS) |
| 7 | - | 3-Cl | CH | 265(x1/2 NDS) |
| 8 | 4-Cl | 2-Cl | N | 152 |

NDS = 1,5-Naphthalindisulfonsäure

Entsprechend Beispiel 1 können die in der folgenden Tabelle 2 aufgeführten Ausgangsprodukte der Formel (II) erhalten werden:

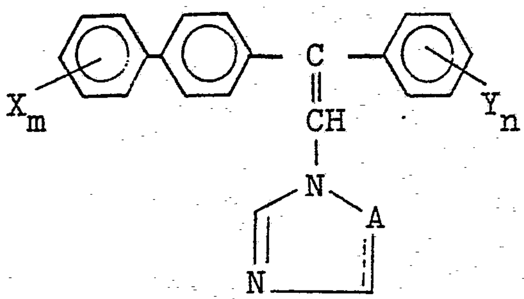

(II)

| Bsp.Nr. | $X_m$ | $Y_n$ | A | Schmelzpunkt (°C) |
|---|---|---|---|---|
| (II-2) | 4-Cl | 2-Cl | CH | 174 |
| (II-3) | - | 2-F | CH | Oel |
| (II-4) | - | 3-Cl | CH | 211 |
| (II-5) | - | 2-F | CH | 325(Zers.) (x 1/2 NDS) |
| (II-6) | 4-Cl | 2-Cl | N | 170 |
| (II-7) | - | 4-Cl | CH | $n_D^{20}$ = 1,6350 |

Verwendungsbeispiele

In dem nachfolgenden Beispiel werden die nachstehend angegebenen Verbindungen als Vergleichssubstanzen eingesetzt:

(A) 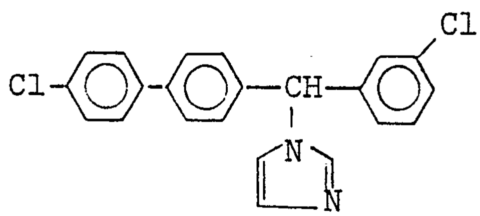

(B) 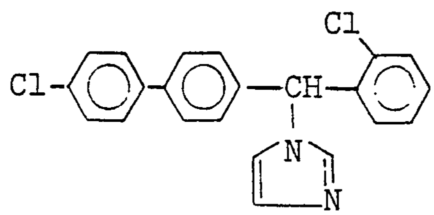

(C) 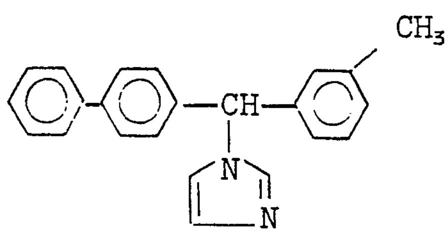

(D) 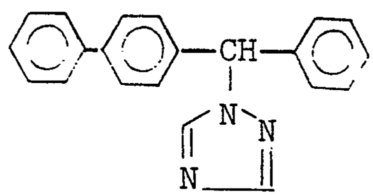

## Beispiel A

### Antimykotische in-vitro-Wirksamkeit

### Versuchsbeschreibung:

Die in-vitro-Prüfung wurde im Reihenverdünnungstest mit Keiminokula von durchschnittlich 5 x 10 Keimen/ml Substrat durchgeführt. Als Nährmedium dienten

a) für Dermatophyten und Schimmelpilze:
   Sabourand's milieu d'épresive

b) für Hefen:
   Isotonic Sensitest-Broth von Oxid.

Die Bebrütungstemperatur betrug 28°C; Bebrütungsdauer war 24 Stunden bei Hefen und 96 Stunden bei Dermatophyten und Schimmelpilzen.

In diesem Test zeigen z.B. die Verbindungen gemäß Herstellungsbeispiel 5,6,7 und 8 eine sehr gute Wirkung, die derjenigen der aus dem Stand der Technik bekannten Verbindungen (A), (B), (C) und (D) überlegen ist.

Patentansprüche

1. Biphenylyl-azolylethan-derivate der allgemeinen Formel

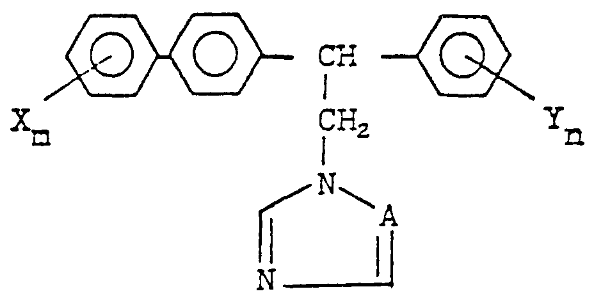

(I)

in welcher

A für die CH-Gruppe oder ein Stickstoffatom steht,

X für Halogen, Alkyl, Halogenalkyl, Alkoxy, Alkylthio, Nitro oder Cyano steht,

Y für Halogen, Alkyl, Halogenalkyl, Alkoxy, Alkylthio, Nitro, Cyano oder gegebenenfalls substituiertes Phenyl steht und

m und n für 0, 1, 2 oder 3 stehen,

und deren physiologisch verträglichen Säureadditions-Salze.

2. Biphenylyl-azolylethan-derivate der allgemeinen Formel

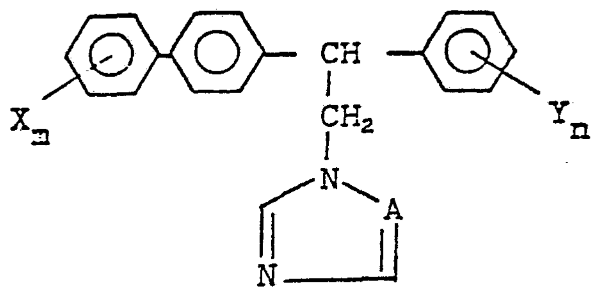

(I)

in welcher

A, m und n die oben angegebene Bedeutung haben,

X für Halogen, geradkettiges oder verzweigtes Alkyl mit 1 bis 4 Kohlenstoffatomen, Halogenalkyl mit 1 bis 4 Kohlenstoff- und 1 bis 5 gleichen oder verschiedenen Halogenatomen, wobwi als Halogene vorzugsweise Fluor und Chlor stehen, ferner für Alkoxy und Alkylthio mit jeweils 1 bis 4 Kohlenstoffatomen, sowie Nitro und Cyano steht und

Y für die für X angegebenen Bedeutungen und außerdem für durch X substituiertes Phenyl steht,

und deren physiologisch verträglichen Säureadditionssalze.

3. Biphenylyl-azolylethan-derivate der allgemeinen Formel

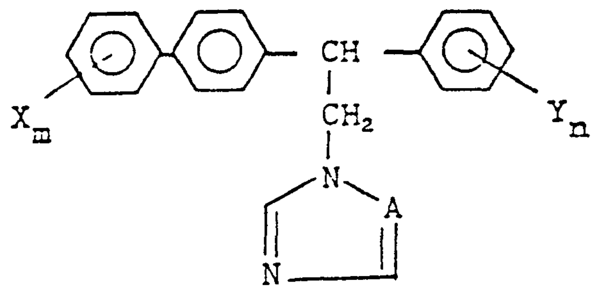

(I)

in welcher

A, m und n die oben angegebene Bedeutung haben,

X für Fluor, Chlor, Brom, Methyl, Ethyl, Isopropyl, tert.-Butyl, Trifluormethyl, Methoxy, Ethoxy, Methylthio, Ethylthio, Nitro oder Cyano steht;

Y für die für X angegebenen Bedeutungen und außerdem für gegebenenfalls durch Fluro, Chlor, Methyl, tert.-Butyl, Trifluormethyl, Nitro oder Cyan substituiertes Phenyl steht,

und deren physiologisch verträglichen Säureadditionssalze.

4. 1-(4-Biphenylyl)-1-(2-chlorphenyl)-2-(imidazol-1-yl)-ethan.

5. 1-(4-Biphenylyl)-1-(2-flurophenyl)-2-(imidazol-1-yl)-ethan.

6. 1-(4-(Biphenylyl)-1-(3-chlorphenyl)-2-(imidazol-1-yl)-ethan.

7. 1-(4'-chlorphenyl-4-phenyl)-1-) 2-chlorphenyl)-2-(1,2,4-triazol-1-yl)-ethan.

8. Verfahren zur Herstellung von Biphenylyl-azolylethanen der allgemeinen Formel I, dadurch gekennzeichnet, daß man Biphenylyl-azolylethylen-derivate der allgemeinen Formel

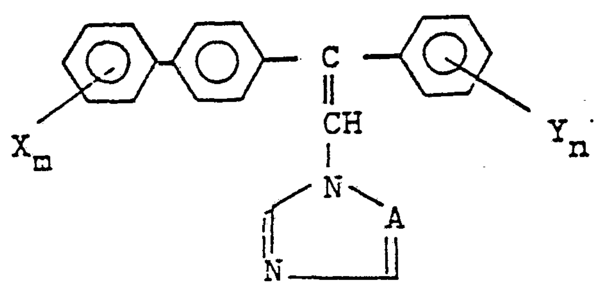

(II)

in welcher

A, X, Y, m und n die oben angegebene Bedeutung haben,

in üblicher Weise mit Wasserstoff in Gegenwart eines Katalysators und gegebenenfalls in Gegenwart eines Verdünnungsmittels hydriert.

9. Arzneimittel, gekennzeichnet durch einen Gehalt an mindestens einem Biphenylyl-azolylethan der allgemeinen Formel I und/oder deren physiologisch verträglichen Säureadditionssalze.

10. Verfahren zur Behandlung von Mykosen, dadurch gekennzeichnet, daß man Biphenylyl-azolylethanderivate der allgemeinen Formel I Menschen oder Tieren appliziert, die an Mykosen erkrankt sind.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung: EP 81 10 2165.8

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | DE - A1 - 2 851 059 (BAYER AG)<br>* Ansprüche 1, 3 *<br>-- | 1,9 |
| | EP - A1 - 0 003 796 (BAYER AG)<br>* Anspruch 3; Beispiel 1 *<br>-- | 1,9 |
| D | DE - A1 - 2 461 406 (BAYER AG)<br>* Ansprüche 1, 3 *<br>---- | 1,9 |

KLASSIFIKATION DER ANMELDUNG (Int.Cl.3)

C 07 D 233/56
C 07 D 249/08
A 61 K 31/41
A 61 K 31/415

RECHERCHIERTE SACHGEBIETE (Int. Cl.3)

A 61 K 31/00
C 07 D 233/56
C 07 D 249/08

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: --
Nicht recherchierte Patentansprüche: 10 . Art. 52(4)
Grund für die Beschränkung der Recherche:

Verfahren zur therapeutischen Behandlung des menschlichen oder tierischen Körpers

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 22-06-1981 | FROELICH |